# DEMANDE DE BREVET EUROPEEN

(11) **EP 1 559 406 A1**
(43) Date de publication de la demande: **03.08.2005**
(21) Numéro de dépôt: 05290084.2
(22) Date de dépôt: 14.01.2005
(51) Int. Cl.: A61K 7/13

(54) **Composition de teinture des fibres kératiniques contenant une alcool oxydase et un agent tensio-actif non-ionique dérivé de sucre, procédé mettant en oeuvre cette composition**

(30) Priorité: 28.01.2004 FR 0400780
(71) Demandeur: L'OREAL, 75008 Paris (FR)
(72) Inventeur: Plos, Grégory, Tokyo 158-0097 (JP)
(74) Mandataire: Casalonga, Axel

(57) **Abrégé**

La présente demande concerne une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un agent tensio-actif. Cette demande concerne encore un procédé de teinture des fibres kératiniques qui consiste à appliquer cette composition ainsi qu'un « kit » de teinture.

## Description

La présente invention a pour objet une composition pour la teinture des fibres kératiniques, en particulier des fibres kératiniques humaines, telles que les cheveux, contenant, dans un milieu approprié pour la teinture, au moins un précurseur de colorant d'oxydation, au moins une enzyme alcool oxydase, au moins un substrat pour ladite enzyme et au moins un agent tensio-actif non-ionique.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi la demande de brevet FR 2769219 décrit l'utilisation d'une enzyme uricase et de son substrat l'acide urique en coloration d'oxydation pour la teinture de fibres kératiniques. La demande EP-A-0 310 675 décrit l'utilisation de précurseur de colorant d'oxydation de type benzénique en association avec des enzymes telles que la pyranose-oxydase, la glucose-oxydase. Plus récemment, la demande de brevet FR 2.833.492 décrit l'utilisation de l'enzyme alcool-oxydase en tant que seule enzyme dans une composition de coloration d'oxydation pour la teinture de fibres kératiniques.

Le but de la présente invention est de fournir de nouvelles compositions pour la teinture des fibres kératiniques par coloration d'oxydation homogènes et stables, utilisant un système oxydant différent du peroxyde d'hydrogène.

De manière avantageuse et inattendue, la demanderesse vient maintenant de découvrir qu'il est possible d'atteindre ce but en utilisant au moins un précurseur de colorant d'oxydation, au moins une enzyme alcool oxydase, au moins un substrat pour ladite enzyme et au moins un agent tensio-actif non-ionique dérivé de sucre dans une composition pour la teinture des fibres kératiniques, en particulier des fibres kératiniques humaines telles que les cheveux.

La composition selon l'invention permet l'obtention de formulations particulièrement homogènes qui, une fois appliquées, respectent la nature des fibres kératiniques et ne présentent pas les problèmes de solubilisation et de cristallisation rencontrés notamment avec le système acide urique/ uricase. La demanderesse a aussi noté que la stabilité de la composition selon l'invention et en particulier la stabilité de l'enzyme alcool oxydase de cette composition est améliorée.

Les compositions selon la présente invention présentent l'avantage de conduire à l'obtention de teintures de couleurs puissantes, peu sélectives et résistantes, ces compositions sont capables d'engendrer des nuances variées de couleur intense et uniforme, sans dégradation significative du cheveu. En outre, on a noté que l'utilisation d'une telle composition améliore la tenue des cheveux permanentés et diminue la porosité du cheveu.

D'autres caractéristiques, aspects, objets et avantages de la présente invention apparaîtront encore plus clairement à la lecture de la description et des exemples qui suivent.

L'agent tensio-actif non-ionique dérivé de sucre utilisable selon la présente invention est choisi dans le groupe constitué par :
1- les alkylpolyglucosides;
2- les esters d'acides gras de sucre ou d'alkylsucre;
3- les sucramides gras ;
4- leurs mélanges.

Les agents tensio-actifs non-ioniques de type alkylglucoside (1) utilisés dans la présente invention sont des produits bien connus en soi qui peuvent être plus particulièrement représentés par la formule générale (I) suivante :

R₁-O-(R₂-O)ₐ-(L)_{b} **(I)**

dans laquelle, R¹ désigne un radical alkyle et/ou alcényle linéaire ou ramifié comportant environ de 8 à 24 atomes de carbone, un radical alkylphényle dont le groupe alkyle linéaire ou ramifié comporte environ de 8 à 24 atomes carbone, R² désigne un radical alkylène comportant de 2 à 4 atomes de carbone, L désigne un sucre réduit comportant de 5 à 6 atomes de carbone, a désigne une valeur allant de 0 à 10, et b désigne une valeur allant de 1 à 15.

Des alkylpolyglucosides préférés selon la présente invention sont des composés de formule (I) dans laquelle R¹ désigne plus particulièrement un radical alkyle et/ou alcényle linéaire ou ramifié comportant de 9 à 14 atomes de carbone, a désigne une valeur allant de 0 à 3 et plus particulièrement encore égale à zéro, L désigne le glucose, le fructose ou le galactose. Le degré de polymérisation (S) du saccharide, i.e. la valeur de b dans la formule (I), peut aller de 1 à 15. Selon l'invention, on préfère les sucres réduits contenant 80 %, ou plus, de sucres dont le degré de polymérisation (S) prend une valeur allant de 1 à 4.

Des composés de formule (I) sont notamment représentés par les produits vendus par la société HENKEL sous la dénomination APG, tels que les produits APG 300, APG 350, APG 500, APG 550, APG 625, APG base 10-12; les produits vendus par la société SEPPIC sous les dénominations TRITON CG 110 (ou ORAMIX CG 110) et TRITON CG 312 (ou ORAMIX NS 10); ceux vendus par la société B.A.S.F. sous la dénomination LUTENSOL GD 70; ceux vendus par la société HENKEL sous les dénominations PLANTAREN 1200, PLANTAREN 1300, PLANTAREN 2000, et PLANTACARE 2000, PLANTACARE 818, PLANTACARE 1200.

Les agents tensio-actifs non-ioniques de type ester d'acide gras de sucre ou d'alkylsucre (2) utilisés dans la présente invention sont des esters d'acides gras en C₄-C₂₂ de sucre ou d'alkylsucre dont on peut notamment citer :
(2)(a) les esters d'alkyl(C₁-C₄)glucoside, tels que :
   - le monostéarate de méthylglucoside, comme le produit vendu sous la dénomination GRILLOCOSE IS par la société GRILLOWERKE;
   - le sesquistéarate de méthylglucoside, comme le produit vendu sous la dénomination GLUCATE SS par la société AMERCHOL;
   - le décanoate d'éthyl-6-glucoside, comme le produit vendu sous la dénomination BIOSURF 10 par la société NOVO;
   - le mélange de mono et dicocoate (82/7) d'éthyl-6-glucoside, comme le produit vendu sous la dénomination BIOSURF COCO par la société NOVO;
   - le mélange de mono et dilaurate (84/8) d'éthyl-6-glucoside, comme le produit vendu sous la dénomination BIOSURF 12 par la société NOVO;
   - les monoesters d'acide gras en C₁₂-C₁₈ du butylglucoside, tels que le monococoate de butylglucoside, comme le produit vendu sous les dénominations REWOPOL V3101 ou REWOSAN V3101 et le monococoate de butylglucoside polyoxyéthyléné avec 3 moles d'oxyde d'éthylène, comme le produit vendu sous la dénomination REWOPOL V3122 par la société REWO.
(2)(b) les esters de glucose tels que:
   - l'O-hexadécanoyl-6-aD-glucose, l'O-octanoyl-6-D-glucose, l'O-oleyl-6-D-glucose, l'O-linoleyl-6-D-glucose, qui sont des composés connus pouvant être préparés, par exemple, à partir du chlorure d'acide correspondant et du D-glucose, selon la méthode décrite par E. REINEFELD et Coll. ; "Die Stärke" n°6 - pages 181-189, - 1968.
(2)(c) les monoesters de saccharose, tels que:
   - le monolaurate de saccharose, comme le produit vendu sous la dénomination GRILLOTEN LES 65, et,
   - le monococoate de saccharose vendu sous la dénomination GRILLOTEN LES 65K, par la société GRILLO-WERKE.

Les agents tensio-actifs non-ioniques de type sucramide gras (3) utilisés dans la présente invention sont des composés comportant au moins une fonction amide et incluant au moins une portion sucre ou dérivée de sucre et au moins une chaîne grasse; de tels composés peuvent, par exemple, résulter de l'action d'un acide gras ou d'un dérivé d'acide gras sur la fonction amine d'un aminosucre, ou de l'action d'une amine grasse sur un sucre comportant une fonction acide carboxylique (libre ou sous forme de lactone) ou dérivée d'acide carboxylique ou encore une fonction carbonyle, et ceci éventuellement en présence de co-réactifs adéquats.

Les sucramide gras (3) utilisés sont choisis de préférence parmi:
(3)(a) les aldonamides N-substitués et,
(3)(b) les amides d'acides gras polyhydroxylés ou leurs mélanges.
   (3)(a) Les aldonamides N-substitués utilisables selon l'invention peuvent être choisis parmi ceux décrits dans la demande de brevet EP-A-550 106 dont le contenu fait partie intégrante de la présente description. Parmi ceux-ci, on peut citer :
      - les lactobionamides N-substitués, les maltobionamides N-substitués, les cellobionamides N-substitués, les mellibionamides N-substitués et les gentiobionamides N-substitués tels que :
         (i) les N-alkyl lactobionamides, N-alkyl maltobionamides, les N-alkyl cellobionamides, les N-alkyl mellibionamides ou les N-alkyl gentiobionamides mono- ou disubstitués par un groupe hydrocarboné aliphatique, linéaire ou ramifié, saturé ou insaturé, pouvant contenir des hétéroatomes ayant de préférence jusqu'à 36 atomes de carbone, plus préférentiellement jusqu'à 24 atomes de carbone et encore plus particulièrement de 8 à 18 (par exemple méthyle, éthyle, amyle, hexyle, heptyle, nonyle, décyle, undécyle, dodecyle, tridécyle, tétradécyle, pentadécyle, hexadécyle, heptadécyle, octadécyle ; allyle, undécenyle, oléyle, linoléyle, propényle, heptènyle), par un groupe hydrocarboné aromatique (par exemple benzyle, aniline, benzyle substitué, phényléthyle, phénoxyéthyle, vinylbenzyle) ou des groupes cycloaliphatiques (par exemple cyclopentyle, cyclohexyle) ;
         (ii) les esters de N-lactobionyl aminoacide, où l'aminoacide peut désigner notamment : alanine, valine, glycine, lysine, leucine, arginine, acide aspartique, acide glutamique, thréonine, serine, cystéine, histidine, tyrosine, méthionine ou bien peut être choisi par exemple parmi la β-alanine, la sarcosine, l'acide gamma-aminobutyrique, l'ornithine, la citrulline ou leurs équivalents ; lesdits esters de N-lactobionyl aminoacides étant monosubstitués par un groupe de formule (II) : 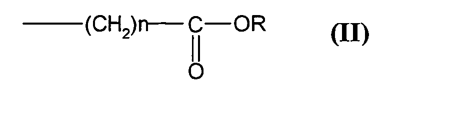 où R est un groupe hydrocarboné aliphatique pouvant contenir jusqu'à 36 atomes de carbones et n est un entier supérieur à 1, ainsi que les esters de N-maltobionyl aminoacide, les esters de N-mellibionyl aminoacide, les esters de N-cellobionyl aminoacide, les esters de N-gentiobionyl aminoacide correspondants,
         (iii) les N-(alkyloxy)alkyl-lactobionamides mono ou disubstitués par un groupe
            -(CH₂)ₙOR' où R' est un groupe hydrocarboné aliphatique, aromatique ou cycloaliphatique tel que défini dans le paragraphe (i) ;
         (iv) les N-(polyalkyloxy)alkyl lactobionamides, les N-(polyalkyloxy)alkyl maltobionamides, les N-(polyalkyloxy)alkyl cellobionamides, les N-(polyalkyloxy)alkyl mellibionamides ou les N-(polyalkyloxy)alkyl gentiobionamides qui sont mono ou disubstitués par un groupe

            -R₁-(OR₁)ₙR₁R₂
         où R₁ est un groupe alkylène tel que éthylène, propylène, ou leurs mélanges, n est un entier supérieur à 1, R₂ est un groupe lactobionamide, maltobionamide, cellobionamide, melliobionamide ou gentiobionamide.
   (3)(b) Les amides gras polyhydroxylés conformes à la présente invention sont choisis de préférence parmi ceux décrits dans le brevet EP-B-550 656 dont le contenu fait partie intégrante de la description et répondant à la formule (III) suivante : 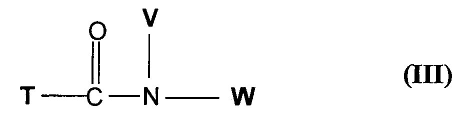 dans laquelle,
      T désigne un groupe hydrocarboné en C₅-C₃₁, de préférence une chaîne alkyle ou alcényle linéaire en C₇-C₁₅ ou leurs mélanges ;
      V désigne un atome d'hydrogène, un radical hydrocarboné en C₁-C₄, 2-hydroxy éthyle, 2-hydroxypropyle ou leurs mélanges, de préférence un alkyle en C₁-C₄ tel que méthyle, éthyle, propyle, isopropyle, N-butyle et plus particulièrement méthyle ;
      W désigne un groupe polyhydroxyhydrocarboné ayant une chaîne linéaire hydrocarbonée avec au moins 3 groupes hydroxyles directement liés à la chaîne, ou un dérivé alcoxylé dudit groupe (de préférence éthoxylé ou propoxylé).
      W est de préférence un dérivé de sucre réducteur obtenu par réaction d'amination réductrice et plus préférentiellement un groupe glycityle. Parmi les sucres réducteurs, on peut citer le glucose, le maltose, le lactose, le galactose, le mannose, et le xylose.
      Plus préférentiellement encore, W est choisi parmi les groupes de formules suivantes :

      -CH₂-(CHOH)ₙ-CH₂OH ;

      -CH-(CH₂OH)-(CHOH)ₙ₋₁-CH₂OH ;

      -CH₂-(CHOH)₂(CHOR')(CHOH)-CH₂OH dans lesquelles n est un nombre entier allant de 3 à 5, R' est hydrogène, un monosaccharide cyclique ou aliphatique ou l'un de ses dérivés alcoxylés,
      et parmi lesquels on préfère encore un groupe glycityle dans lequel n est égal à 4, et en particulier le groupe -CH₂-(CHOH)₄-CH₂OH.
      Le groupe T-CON= peut être par exemple cocamide, stéaramide, oléamide, lauramide, myristiramide, capricamide, palmitamide, amide de suif.

Selon la présente invention, on préfère utiliser plus particulièrement à titre d'agents tensio-actifs non-ioniques dérivés de sucre, les alkylpolyglucosides (1).

Le ou les agents tensio-actifs non-ioniques dérivés de sucre utilisés selon l'invention, représentent de préférence de 0,05 à 30 % en poids environ du poids total de la composition tinctoriale et encore plus préférentiellement de 0,1 à 15 % en poids environ de ce poids.

Dans le cadre de la présente invention, les enzymes alcool oxydases utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes sont choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

De préférence l'enzyme utilisée dans la composition selon l'invention est une alcool primaire oxydase (EC1.1.3.13).

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples, l'enzyme alcool oxydase peut être issue d'une des espèces *suivantes : Rhodococcus erythropolis, Pseudomonas pseudoalcaligenes* qui sont des bactéries, *Aspergillus niger, Kamagataella pastoris, Phanerochaete chrysosporium, Polyporus obtusus, Hansenula polymorpha, Poria contigua, Penicillium simplicissimum, Pleurotus pulmonarius* (champignons), *Pichia sp. (pastoris, methanolica, angusta)* et *Candida sp. (boidinii, albicans,* *tropicalis)* (levures), *Pinus strobus* qui est une espèce d'origine végétale, *Gastropode mollusc, Manduca sexta* qui sont d'origine animale.

De préférence l'enzyme utilisée dans la composition selon l'invention est une alcool oxydase provenant de *Pichia pastoris*.

Généralement, la concentration en enzyme alcool oxydase utilisée dans la composition tinctoriale est comprise 0,05 % et 20 % en poids par rapport au poids total de ladite composition, de préférence comprise entre 0,1 et 10 %, et de manière encore plus préférée entre 0,5 et 8 % en poids par rapport au poids de cette composition.

L'activité enzymatique des enzymes alcool oxydases utilisées conformément à l'invention peut être définie à partir de l'oxydation du donneur en condition aérobie. Unité U correspond à la quantité d'enzyme conduisant à la génération de 1 µmole de peroxyde d'hydrogène par minute à un pH donné et à une température de 25°C.

De façon préférentielle la quantité d'alcool oxydase est comprise entre 10³U et 10⁵ U, de préférence entre 2.10³U et 5.10⁴ U pour 100g de composition tinctoriale.

Le ou les substrats pour ladite enzyme dans les compositions de l'invention sont encore appelés donneurs pour l'enzyme. Le substrat pour l'enzyme est de préférence un alcool choisi parmi les alcools primaires, secondaires, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4-méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique
ou aromatique peut notamment être un précurseur de colorant d'oxydation ou encore un adjuvant cosmétiquement acceptable par un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture au moins les composés suivants : un précurseur de colorant d'oxydation ; au moins une enzyme alcool oxydase ; au moins un substrat, portant une fonction alcool, pour ladite enzyme et au moins un tensioactif non-ionique dérivé de sucre, ledit substrat pouvant être substitué (c'est-à-dire remplacé) en tout ou en partie par le précurseur d colorant d'oxydation dans le cas où il porte au moins une fonction alcool aliphatique ou aromatique.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration en substrat pour l'enzyme est comprise entre 0,01% et 60% en poids par rapport au poids total de la composition et de préférence comprise entre 0,05% et 30% en poids par rapport au poids total de la composition.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène, la 3-hydroxy 1-(4' aminophenyl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2,6-dichlorophénol le 4-amino-6[(5'-amino-2'-hydroxy-3'-méthyl)phénylméthyl] 2-méthylphénol, le bis-(5'-amino-2'-hydroxyl)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation est comprise entre 0,0001 et 20 %, de préférence entre 0,005 à 6 % en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20 %, de préférence entre 0,005 à 6 % en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, ces colorants directs additionnels sont choisis parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des tensioactifs autres que ceux de l'invention, des agents de conditionnement tels que par exemple des silicones volatiles ou non volatiles, modifiées ou non modifiées, des polymères cationiques, des cations, des agents filmogènes, des céramides, des agents conservateurs, des agents opacifiants, des vitamines ou provitamines et polymères épaississants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol, l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 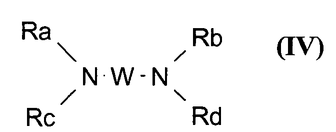 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides épaissis, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, la durée de cette application étant la durée suffisante pour développer la coloration désirée.

La couleur est alors révélée par la mise en présence de l'enzyme alcool oxydase et de son substrat en présence d'oxygène.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins un agent tensio-actif non-ionique, l'ensemble est alors stocké sous forme anaérobie, exempte d'oxygène gazeux.

Selon une variante, le procédé comporte une étape préliminaire consistant à stocker sous forme séparée, d'une part une composition (A) comprenant, dans un milieu approprié pour la teinture des fibres kératiniques, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins un agent tensio-actif non-ionique dérivé de sucre puis à procéder au mélange des compositions (A) et (B) au moment de l'emploi avant d'appliquer ce mélange sur les fibres kératiniques.

Selon une variante de l'invention, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour ladite enzyme et au moins un agent tensio-actif non-ionique dérivé de sucre et d'autre part, une composition (B) renfermant, dans un milieu approprié pour la teinture des fibres kératiniques, au moins une enzyme alcool oxydase, puis à procéder au mélange des compositions (A) et (B) au moment de l'emploi avant d'appliquer ce mélange sur les fibres kératiniques.

La couleur peut être révélée à pH acide, neutre ou al calin. Dans le cas où le procédé est mis en oeuvre au moyen d'une composition (A) comprenant au moins un précurseur de colorant d'oxydation, au moins un substrat pour ladite enzyme et au moins un agent tensio-actif non-ionique dérivé de sucre et d'une composition (B) renfermant au moins une enzyme alcool oxydase, l'enzyme peut être ajoutée à la composition de l'invention juste au moment de l'emploi ou peut être mis en oeuvre à partir d'une composition la contenant, appliquée simultanément ou séquentiellement à la composition de l'invention.

Cette composition (dite composition oxydante) peut également renfermer divers adjuvants utilisés classiquement dans les compositions pour la teinture des cheveux et tels que définis précédemment.

Le pH de la composition B dite oxydante est tel qu'après mélange avec la composition tinctoriale A, le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

On prépare la composition suivante :

| **Constituant** | **Composition 1** |
|---|---|
| Alkylpolyglucoside (ORAMIX CG110 - SEPPIC) | 8 g |
| Ethanol | 25 g |
| Paraphénylènediamine | 3.10⁻³ mole |
| Métaaminophénol | 3.10⁻³ mole |
| Alcool oxydase | 20000 unités |
| 2-amino-2-méthyl-1-propanol | qs pH 7 |
| Eau distillée | qsp 100 g |

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

On obtient une coloration résistante et homogène dans des nuances vert kaki.

## Revendications

1. Composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un agent tensio-actif non ionique dérivé de sucre, ledit substrat pouvant être substitué en tout ou en partie par le précurseur de colorant d'oxydation dans les cas où il porte au moins une fonction alcool aliphatique ou aromatique.

2. Composition selon la revendication 1 telle que l'agent tensio-actif non-ionique utilisable selon la présente invention est choisi dans le groupe constitué par les alkylpolyglucosides, les esters d'acides gras de sucre ou d'alkylsucre, les sucramides gras, et leurs mélanges.

3. Composition selon la revendication 2 telle que l'agent tensio-actif non-ionique est un alkylglucoside de formule (I) suivante :
R₁ -O-(R₂-O)ₐ-(L)_{b} **(I)**
dans laquelle, R¹ désigne un radical alkyle et/ou alcényle linéaire ou ramifié comportant environ de 8 à 24 atomes de carbone, un radical alkylphényle dont le groupe alkyle linéaire ou ramifié comporte environ de 8 à 24 atomes carbone, R² désigne un radical alkylène comportant de 2 à 4 atomes de carbone, L désigne un sucre réduit comportant de 5 à 6 atomes de carbone, a désigne une valeur allant de 0 à 10, et b désigne une valeur allant de 1 à 15.

4. Composition selon la revendication 3 telle que l'agent tensio-actif non-ionique est un alkylglucoside de formule (I) dans laquelle R¹ désigne un radical alkyle et/ou alcényle linéaire ou ramifié comportant de 9 à 14 atomes de carbone, a désigne une valeur allant de 0 à 3 et de préférence égale à zéro, b désigne une valeur allant de 1 à 15 et de préférence de 1 à 4, L désigne le glucose, le fructose ou le galactose.

5. Composition selon la revendication 2 telle que l'agent tensio-actif non-ionique est un ester d'acide gras en C₄-C₂₂ de sucre ou d'alkylsucre choisi dans le groupe constitué par les esters d'alkyl(C₁-C₄)glucoside, les esters de glucose et les monoesters de saccharose.

6. Composition selon la revendication 5 telle que l'agent tensio-actif non-ionique est un ester d'alkyl(C₁-C₄)glucoside choisi dans le groupe constitué par le monostéarate de méthylglucoside, le sesquistéarate de méthylglucoside, le décanoate d'éthyl-6-glucoside, le mélange de mono et dicocoate (82/7) d'éthyl-6-glucoside, le mélange de mono et dilaurate (84/8) d'éthyl-6-glucoside et les monoesters d'acide gras en C₁₂-C₁₈ du butylglucoside.

7. Composition selon la revendication 5 telle que l'agent tensio-actif non-ionique est un ester de glucose choisi dans le groupe constitué par l'O-hexadécanoyl-6-αD-glucose, l'O-octanoyl-6-D-glucose, l'O-oleyl-6-D-glucose, l'O-linoleyl-6-D-glucose.

8. Composition selon la revendication 5 telle que l'agent tensio-actif non-ionique est un monoester de saccharose choisi dans le groupe constitué par le monolaurate de saccharose et le monococoate de saccharose.

9. Composition selon la revendication 2 telle que l'agent tensio-actif non-ionique est un sucramide gras choisi dans le groupe constitué par les aldonamides N-substitués et les amides d'acides gras polyhydroxylés et leurs mélanges.

10. Composition selon la revendication 9 telle que l'agent tensio-actif non-ionique est un aldonamide N-substitué choisi dans le groupe constitué par les lactobionamides N-substitués, les maltobionamides N-substitués, les cellobionamides N-substitués, les mellibionamides N-substitués et les gentiobionamides N-substitués.

11. Composition selon la revendication 10 telle que l'agent tensio-actif non-ionique est un aldonamide N-substitué choisi dans le groupe constitué par :
- les N-alkyl lactobionamides, les N-alkyl maltobionamides, les N-alkyl cellobionamides, les N-alkyl mellibionamides, les N-alkyl gentiobionamides mono- ou disubstitués par un groupe hydrocarboné aliphatique, linéaire ou ramifié, saturé ou insaturé, pouvant contenir des hétéroatomes ayant de préférence jusqu'à 36 atomes de carbone, plus préférentiellement jusqu'à 24 atomes de carbone et encore plus particulièrement de 8 à 18, par un groupe hydrocarboné aromatique ou des groupes cycloaliphatiques,
- les esters de N-lactobionyl aminoacide, l'aminoacide étant choisi dans le groupe formé par l'alanine, la valine, la glycine, la lysine, la leucine, l'arginine, l'acide aspartique, l'acide glutamique, la thréonine, la serine, la cystéine, la histidine, la tyrosine, la méthionine, la β-alanine, la sarcosine, l'acide gamma-aminobutyrique, l'ornithine, la citrulline ou leurs équivalents ; lesdits esters de N-lactobionyl aminoacides étant monosubstitués par un groupe de formule (II) suivante : 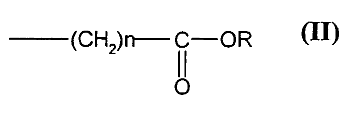
dans lequel R est un groupe hydrocarboné aliphatique pouvant contenir jusqu'à 36 atomes de carbones et n est un entier supérieur à 1, ainsi que les esters de N-maltobionyl aminoacide, les esters de N-mellibionyl aminoacide, les esters de N-cellobionyl aminoacide, les esters de N-gentiobionyl aminoacide correspondants,
- les N-(alkyloxy)alkyl-lactobionamides mono ou disubstitués par un groupe -(CH₂)ₙOR' où R' est un groupe hydrocarboné aliphatique, aromatique ou cycloaliphatique,
- les N-(polyalkyloxy)alkyl lactobionamides, les N-(polyalkyloxy)alkyl maltobionamides, les N-(polyalkyloxy)alkyl cellobionamides, les N-(polyalkyloxy)alkyl mellibionamides ou les N-(polyalkyloxy)alkyl gentiobionamides mono ou disubstitués par un groupe -R₁-(OR₁)ₙR₁R₂ dans lequel R₁ est un groupe alkylène tel que éthylène, propylène, ou leurs mélanges, n est un entier supérieur à 1 et R₂ est un groupe lactobionamide, maltobionamide, cellobionamide, melliobionamide ou gentiobionamide.

12. Composition selon la revendication 9 telle que l'agent tensio-actif non-ionique est un amide gras polyhydroxylé de formule (III) suivante : 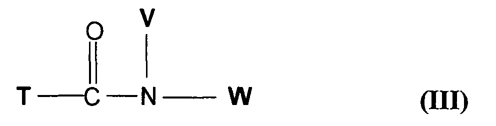 dans laquelle,
T désigne un groupe hydrocarboné en C₅-C₃₁, de préférence une chaîne alkyle ou alcényle linéaire en C₇-C₁₅ ou leurs mélanges ;
V désigne hydrogène, un radical hydrocarboné en C₁-C₄, 2-hydroxy éthyle, 2-hydroxypropyle ou leurs mélanges, de préférence un alkyle en C₁-C₄ et plus particulièrement méthyle ;
W désigne un groupe polyhydroxyhydrocarboné ayant une chaîne linéaire hydrocarbonée avec au moins 3 groupes hydroxyles directement liés à la chaîne, ou un dérivé alcoxylé dudit groupe, de préférence éthoxylé ou propoxylé.

13. Composition selon la revendication 12 telle que l'agent tensio-actif non-ionique est un amide gras polyhydroxylé de formule (III) dans lequel W est un dérivé de sucre réducteur choisi dans le groupe formé par le glucose, le maltose, le lactose, le galactose, le mannose, et le xylose.

14. Composition selon la revendication 13 telle que l'agent tensio-actif non-ionique est un amide gras polyhydroxylé de formule (III) dans lequel W est choisi parmi les groupes de formules suivantes :
-CH₂-(CHOH)ₙ-CH₂OH ;
-CH-(CH₂OH)-(CHOH)ₙ₋₁-CH₂OH ;
ou
-CH₂-(CHOH)₂(CHOR')(CHOH)-CH₂OH
dans lesquelles n est un nombre entier allant de 3 à 5, R' est hydrogène, un monosaccharide cyclique ou aliphatique ou l'un de ses dérivés alcoxylés, de préférence le groupe -CH₂-(CHOH)₄-CH₂OH.

15. Composition selon la revendication 13 telle que l'agent tensio-actif non-ionique est un amide gras polyhydroxylé de formule (III) dans lequel le groupe T-CON= est choisi dans le groupe formé par cocamide, stéaramide, oléamide, lauramide, myristiramide, capricamide, palmitamide et amide de suif.

16. Composition selon l'une quelconque des revendications précédentes telle que le ou les agents tensio-actifs non ioniques dérivés de sucre sont présents en une quantité allant de 0,05 % à 30 % en poids environ du poids total de la composition, de préférence de 0,1 à 15 % en poids.

17. Composition selon l'une des revendications précédentes telle que l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7).

18. Composition selon la revendication 17 telle l'enzyme alcool oxydase est issue d'une des espèces suivantes : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmonarius, Pichia sp. (pastoris, methanolica, angusta)* et *Candida sp. (boidinii, albicans, tropicalis) , Pinus strobus, Gastropode mollusc, Manduca sexta*, de préférence *Pichia pastoris.*

19. Composition selon l'une des revendications 17 ou 18, telle que la concentration en enzyme alcool oxydase est comprise entre 0,05-20 % en poids par rapport au poids total de ladite composition et de préférence comprise entre 0,1 et 10 %, et plus particulièrement entre 0,5-8 % en poids par rapport au poids total de ladite composition.

20. Composition selon l'une des revendications précédentes telle que le substrat pour l'enzyme est un alcool choisi parmi les alcools primaires, secondaires, les alcools à longue chaîne hydrocarbonée et les alcools aromatiques.

21. Composition selon la revendication 20 telle que la concentration en alcool est comprise entre 0,01% et 60% en poids par rapport au poids total de la composition et de préférence comprise entre 0,05% et 30% en poids par rapport au poids total de la composition.

22. Composition selon l'une des revendications précédentes telle qu'elle comprend en tant que précurseur de colorant d'oxydation une base d'oxydation classique choisie parmi les paraphénylènediamines, les bis-phénylalkylènediamines, les para-aminophénols, les ortho-aminophénols, les bases hétérocycliques et leurs sels d'addition.

23. Composition selon la revendication 22 telle que la concentration de la ou des bases d'oxydation est comprise entre 0,0001 et 20% en poids par rapport au poids total de la composition.

24. Composition selon l'une des revendications précédentes telle que la composition comprend en tant que précurseur de colorant d'oxydation un coupleur d'oxydation classique choisi parmi les métaphénylènediamines, les métaaminophénols, les métadiphénols, les coupleurs naphtaléniques, les coupleurs hétérocycliques ainsi que leurs sels d'addition.

25. Composition selon la revendication 24 telle que la concentration du ou des coupleurs est comprise entre 0,0001 et 20 % en poids par rapport au poids total de la composition.

26. Composition selon l'une des revendications précédentes **caractérisée par le fait qu'**elle renferme un ou plusieurs colorants directs naturels ou cationiques.

27. Procédé de teinture des fibres kératiniques et en particulier des fibres kératiniques humaines telles que les cheveux, **caractérisée par le fait qu'**on applique sur lesdites fibres au moins une composition selon l'une des revendications 1 à 26, pendant une durée suffisante pour développer la coloration désirée.

28. Procédé selon la revendication 27, **caractérisé par le fait que** la composition est une composition prête à l'emploi comprenant, dans un milieu approprié pour la teinture des fibres kératiniques, une composition selon l'une des revendications 1 à 26, l'ensemble étant stocké sous forme anaérobie, exempte d'oxygène gazeux.

29. Procédé selon la revendication 28, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins un tensioactif non ionique dérivé de sucre, puis à procéder au mélange des compositions (A) et (B) au moment de l'emploi avant d'appliquer ce mélange sur les fibres kératiniques.

30. Procédé selon la revendication 29, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour l'enzyme alcool oxydase et au moins un tensioactif dérivé de sucre et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

31. Dispositif à plusieurs compartiments ou « Kit » de teinture, **caractérisé par le fait qu'**il comporte un premier compartiment renfermant la composition (A) telle que définie à l'une des revendications 29 ou 30 et un second compartiment renfermant la composition (B) telle que définie à l'une des revendications 29 ou 30.
